# EUROPEAN PATENT APPLICATION

(11) **EP 4 843 601 A1**
(43) Date of publication of application: **25.11.2026**
(21) Application number: 26180399.3
(22) Date of filing: 21.05.2026
(51) Int. Cl.: A61M 25/00

(54) **HOLE CONFIGURATION FOR AN INTERMITTENT CATHETER**

(30) Priority: 23.05.2025 US 202519217989
(71) Applicant: Wellspect AB, 431 21 Mölndal (SE)
(72) Inventor: ENGSTRÖM, Axel, 423 39 Torslanda (SE); DAHLBERG, Niklas, 443 38 Lerum (SE)
(74) Representative: Allen, Caroline Margaret

(57) **Abstract**

An intermittent urinary catheter (100) can include a shaft (102) and a plurality of inlets (106). The shaft (102) can extend between a proximal end portion and a distal end portion. The shaft (102) can define a lumen (104) therethrough. The plurality of inlets (106) can be formed in the distal end portion. Each inlet of the plurality of inlets (106) is in fluidic communication with the lumen (104). A total area of the plurality of inlets (106) can be greater than a cross-sectional area of the lumen (104).

## Description

### TECHNICAL FIELD

Examples described herein generally relate to an Intermittent catheter and more specifically to hole configurations for an intermittent catheter.

### BACKGROUND

Intermittent catheters can provide a means for draining urine from the bladder at regular intervals. Healthcare providers can use these devices for users who have difficulty emptying their bladders naturally due to various medical conditions. Intermittent catheterization can involve inserting a thin, flexible tube through the urethra and into the bladder to allow urine to flow out. Users can remove the catheter after each use, typically performing this procedure several times a day. Users can also use the intermittent catheter without the help of healthcare providers to help them empty their bladders.

These catheters can come in various sizes and materials to suit different user needs. Some intermittent catheters can feature coatings or lubricants to ease insertion and enhance comfort. Healthcare professionals can teach users or caregivers how to properly use and maintain these devices, promoting independence and reducing the risk of complications associated with long-term indwelling catheters.

### SUMMARY

In examples, an intermittent urinary catheter can include a shaft and a plurality of inlets. The shaft can extend between a proximal end portion and a distal end portion. The shaft can define a lumen therethrough. The plurality of inlets can be formed in the distal end portion. Each inlet of the plurality of inlets can be in fluidic communication with the lumen. The plurality of inlets can be arranged in a helical pattern extending from a position adjacent a distal end portion of the shaft toward the proximal end portion.

In examples, an intermittent urinary catheter can include a shaft and a plurality of inlets. The shaft can extend between a proximal end portion and a distal end portion. The shaft can define a lumen therethrough. The plurality of inlets can be formed in the distal end portion. Each inlet of the plurality of inlets can be in fluidic communication with the lumen. A total area of the plurality of inlets can be greater than a cross-sectional area of the lumen.

### BRIEF DESCRIPTION OF THE DRAWINGS

Various examples are illustrated in the figures of the accompanying drawings. Such examples are demonstrative and not intended to be exhaustive or exclusive examples of the present subject matter.
FIG. 1 illustrates an example of an intermittent catheter including a rear view, a front view, a right-side view, and a left-side view.
FIG. 2 illustrates an enlarged example of an intermittent catheter showing an example of an inlet.
FIG. 3 illustrates an example of an intermittent catheter, including a right-side view, a left-side view, a front view, and a rear view.
FIG. 4 illustrates an example of a distal end portion of an example of a catheter.
FIG. 5 illustrates an example of a distal end portion of an example of a catheter.
FIG. 6 illustrates examples of catheters.
FIG. 7 illustrates examples of catheters.

### DETAILED DESCRIPTION

Intermittent catheters can provide an effective means for draining urine from the bladder at regular intervals. However, existing catheter designs can face challenges related to drainage efficiency and user comfort. For example, traditional catheters can require repositioning of the catheter to facilitate complete emptying of the bladder. Traditional intermittent they can be perceived as sharp and can irritate or harm the urethra during insertion or withdrawal, and their inlets can become blocked by tissue that can impede proper urine flow. Additionally, the arrangement of inlets on conventional catheters can cause discomfort during insertion and removal. Intermittent catheters can be designed with smaller inlet areas to help offset some of these issues, but even intermittent catheters with smaller inlet areas can become blocked by large stones or sediments. Therefore, stones or sediment can remain after the bladder is drained.

To address at least these potential issues, the present disclosure relates to an intermittent urinary catheter with an improved inlet configuration. The catheter discussed herein can feature a plurality of inlets arranged in a helical pattern along the distal end portion of the shaft. This helical arrangement can reduce the likelihood of inlet blockage, ensuring more consistent drainage. The catheter can also incorporate inlets with optimized width-to-length ratios, which can enhance fluid flow while maintaining structural integrity. The helical pattern can also improve user comfort during use by spacing out the inlets and not having any inlets aligned circumferentially with adjacent inlets.

The above discussion is intended to provide an overview of subject matter of the present patent application. It is not intended to provide an exclusive or exhaustive explanation of the invention. The description below is included to provide further information about the present patent application.

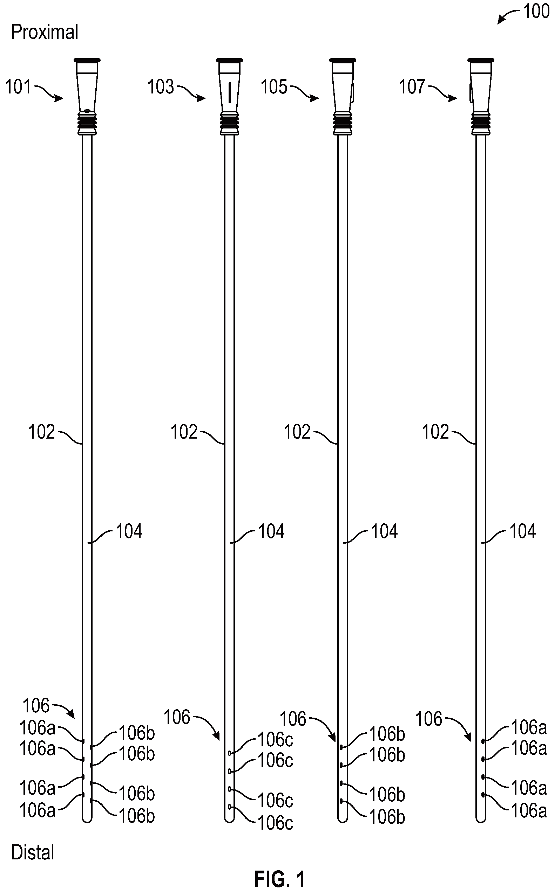

FIG. 1 illustrates an example of an intermittent catheter, e.g., a catheter 100. A rear view 101, a front view 103, a right side view 105, and a left side view 107 of the catheter 100 are each shown in FIG. 1. FIG. 1 also includes visual indicators, Proximal and Distal, to help define Proximal and Distal directions as discussed herein.

The catheter 100 can be an intermittent urinary catheter that can be configured to drain urine from a bladder of a user. The catheter 100 can help fully drain the bladder and minimize the residual amount of urine in the bladder to reduce the risk of urinary tract infections and can help promote healing. In examples, the catheter 100 can be used for users with urinary retention, bladder outlet obstruction, or other conditions that require drainage of the bladder at regular intervals. For example, the catheter 100 can be used in long-term care settings, such as home care settings, nursing homes, rehabilitation centers, or the like. The catheter 100 can include a shaft 102 and a plurality of inlets, e.g., inlets 106.

The shaft 102 can extend between a proximal end portion and a distal end portion. The shaft 102 can be sized, e.g., the length of the shaft 102 can vary from one catheter 100 to another catheter 100, to accommodate users of different sizes or needs. For example, to increase the reach of the catheter 100 for example for users with a penis, the length of the shaft 102 can be longer, and a catheter 100 for users with a vagina can include a shorter shaft 102 to prevent the catheter 100 from extending out of the user. The shaft 102 can define a lumen 104 extending therethrough. The lumen 104 can extend from the proximal end portion to the distal end portion and can be configured to draw fluid from the distal end portion to the proximal end portion.

The inlets 106 can be formed in the distal end portion of the shaft 102. Each inlet of the inlets 106 can be in fluidic communication with the lumen 104. The inlets 106 can be configured to draw fluid, debris, stones, or the like, from within the bladder of the user and into the lumen 104. The inlets 106 can include a first set 106a, a second set 106b, and a third set 106c.

Each inlet of the first set 106a of the inlets 106 can be circumferentially aligned around the shaft 102 with the other inlets of the first set 106a of inlets 106. Each inlet of the second set 106b of the inlets 106 can be circumferentially aligned around the shaft 102 with the other inlets of the second set 106b of inlets 106. Each inlet of the third set 106c of the inlets 106 can be circumferentially aligned around the shaft 102 with the other inlets of the third set 106c of the inlets 106. In examples, the catheter 100 can include a fourth set of inlets. Each inlet of the fourth set of inlets can be circumferentially aligned around the shaft 102 with the other inlets of the fourth set of inlets.

The first set 106a of inlets 106 and the second set 106b of inlets 106 can be circumferentially offset from each other and the second set 106b of inlets 106 and the third set 106c of inlets 106 can be circumferentially offset such that the inlets 106 extend, e.g., from a distal end of the shaft 102 toward the proximal end of the shaft 102 or within the distal end portion of the shaft 102 toward a distal end of the shaft 102, in a helical pattern. The helical pattern can help decrease a probability that the inlets are blocked by tissue, debris, stones, or the like and can adequately drain the bladder of the user. For example, if the inlets were all circumferentially aligned, engagement between tissue, debris, or the like and the aligned inlets can cause the inlets to be blocked. Circumferentially offsetting the inlets can help ensure that at least a portion of the inlets are not blocked and can continue to drain the fluid, debris, stones, or the like from the bladder of the user.

The circumferential offsetting and helical arrangement of the inlets 106 can also enhance user comfort during catheter use. Multiple inlets positioned in close proximity can create increased discomfort for users as the catheter is inserted or removed. The angular split or circumferential offset between sets of inlets can help ensure users do not feel multiple inlets simultaneously during insertion or removal of the catheter 100. When inlets are vertically and horizontally aligned (rather than offset in a helical pattern), users can experience greater discomfort as the aligned inlets create a more noticeable discontinuity along the shaft 102 surface.

Additionally, the spacing between inlets 106 can help maintain the mechanical stability of the shaft 102. Overlapping or closely positioned inlets could negatively affect the structural integrity of the tube. By maintaining appropriate spacing between inlets and distributing them in a helical pattern around the circumference of the shaft 102, the catheter 100 can maintain necessary mechanical properties while still providing adequate drainage capabilities. This arrangement helps balance the need for sufficient inlet area with preservation of shaft structural integrity.

Each of the first set 106a of inlets 106, the second set 106b of inlets 106, and the third set 106c of inlets 106 can include four inlets. Each set, e.g., the first set 106a, the second set 106b, the third set 106c of the inlets 106 can be circumferentially offset 120 degrees from adjacent sets of inlets 106. Each of the first set 106a of inlets 106, the second set 106b of inlets 106, and the third set 106c of inlets 106 can include three inlets. Each set, e.g., the first set 106a, the second set 106b, or the third set 106c, of inlets 106 can be circumferentially offset 120 degrees from adjacent sets of inlets 106.

In examples, the catheter 100 can include a fourth set of the inlets 106. Each inlet of the fourth set of the inlets 106 can be circumferentially aligned around the shaft 102 with the other inlets of the fourth set of inlets. Each of the first set 106a of inlets 106, the second set 106b of inlets 106, the third set 106c of inlets 106, and the fourth set 106d of inlets 106 can include three or more inlets. Each set, e.g., the first set 106a, the second set 106b, the third set 106c, or the fourth set, of inlets 106 can be circumferentially offset 90 degrees from adjacent sets of inlets 106.

In examples, the catheter 100 can improve drainage efficiency and user comfort through the unique inlet configuration. For example, the helical arrangement of the inlets 106 can help decrease the probability of inlet blockage by tissue, debris, or stones, promoting more consistent and adequate drainage of the bladder. By distributing the inlets in multiple sets (106a, 106b, 106c) that can be circumferentially offset from each other, the catheter 100 can maintain necessary mechanical properties while still providing sufficient drainage capabilities. The configuration of the inlets 106 disclosed in FIG. 1 can help balance the need for ample inlet area with preservation of shaft structural integrity. Additionally, the spacing and offset between inlet sets can enhance user comfort during catheter use. By avoiding vertical and horizontal alignment of inlets, users may experience less discomfort during insertion and removal of the catheter 100 because the offset pattern creates less noticeable discontinuities along the shaft surface.

Though the sets are defined as circumferentially aligned inlets, in examples, the sets of inlets can also be defined as being longitudinally aligned and circumferentially spaced from other inlets of the same set. For example, the first set can be the most proximal set, and each inlet of the first set can be longitudinally aligned such that each inlet is the same distance from a distal end of the catheter. Each inlet of the second set of inlets can be longitudinally aligned and circumferentially spaced from other inlets of the second set of inlets and longitudinally offset from the inlets of the first set of inlets. Each inlet of the third set of inlets can be longitudinally aligned and circumferentially spaced from other inlets of the third set of inlets. The inlets of the third set of inlets can be longitudinally offset from the inlets of the first set of inlets and the inlets of the second set of inlets. In examples, each inlet can be longitudinally offset between 0.9 and 1.5 x the length of the inlets. In examples, each inlet can be longitudinally offset, or spaced, between 0.9 and 2 x the length of the inlets. For example, each have around 1 x the length of the inlet between the respective inlet and the next longitudinally spaced inlet.

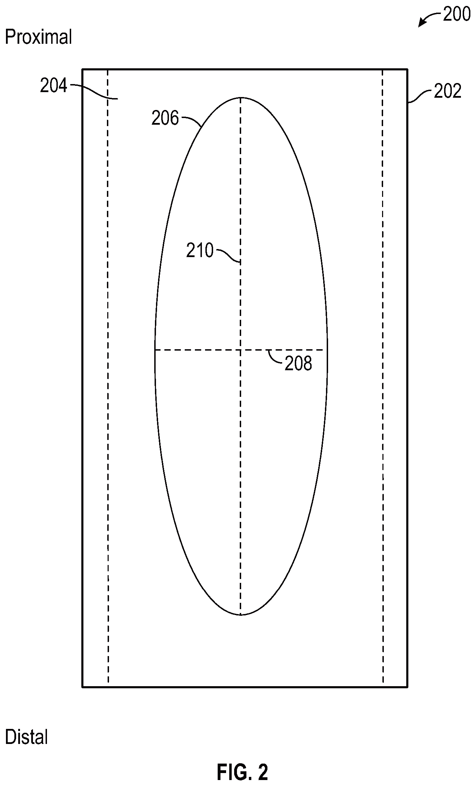

FIG. 2 illustrates an enlarged example of an intermittent catheter, e.g., a catheter 200, illustrating an example of an inlet 206. As discussed herein, each inlet 206 can be sized to help promote the drainage of urine, debris, stones, or the like, from within a bladder of the user. As such, the size, shape, and position of each inlet 206 on the shaft 202 can be adjusted to help promote fluid, debris, stones, or the like to flow into the lumen 204. Each inlet can extend circumferentially to define a width 208 and can extend longitudinally to define a length 210.

The width 208 to length 210 ratio of the inlet 206 can be adjusted to alter the cross-sectional area of the inlet 206. In examples, the inlet 206 can include a width 208 to length 210 ratio of 1:3. In examples, the inlet 206 can include a width 208 to length 210 ratio between 1:1 and 1:4. In examples, a total inlet area, e.g., a total cross sectional area of each of the inlet 206 of the plurality of the inlets, can be greater than a cross-sectional area of the lumen 204. In examples, the total inlet area can be at least twice the cross-sectional area of the lumen 204. The width 208 to length 210 ratio and the overall inlet area can help ensure adequate drainage from the bladder into the lumen 204 and out of the user. For example, increasing the inlet area or the width 208 to length 210 ratio can increase an amount of fluid that can flow into the lumen 204 from the bladder of the user.

The overall shape or profile of the inlet 206 can also be adjusted. As shown in FIG. 1, the inlets 106 can include a generally circular profile, and as shown in FIG. 2, the inlet 206 can include a generally oval profile. In examples, the inlets can include a slot, square, oblong, slit, or any other shape profile to facilitate the draining of fluid, debris, or other bodily tissues or fragments from within the bladder of the user.

In examples, a catheter can include inlets of altering sizes, shapes, or profiles. For example, a set of inlets can include varying shapes, sizes, or profiles within a set. In examples, the plurality of inlets can include different sets where each set has an inlet including a unique shape, size, profile, as compared to the other sets of inlets. In yet another example, the shape, size, or profile of the inlets can change as the inlets extend distally down the shaft.

In examples, the catheter 200 demonstrates how the size, shape, and position of each inlet on the shaft 202 can be designed to promote efficient drainage of urine, debris, and stones from the bladder. For example, by adjusting the width-to-length ratio of the inlet 206, the catheter design can balance the need for adequate drainage while maintaining the structural integrity of the shaft. In examples, a width-to-length ratio between 1:1 and 1:4 can help achieve this balance. Furthermore, the total inlet area can be designed to be greater than the cross-sectional area of the lumen 204, potentially even twice as large or more, to ensure sufficient fluid flow. The design of the inlet 206, as shown at least in FIG. 2, can help address issues of blockage and inadequate drainage common in traditional catheter designs, while also maintaining the overall structural stability of the catheter. By optimizing these parameters, the catheter 200 can offer improved drainage efficiency and potentially enhance user comfort during use.

In examples, each inlet can include a cross-sectional area greater than 0.5 mm². Each inlet can include a cross-sectional area between 0.5 mm² and 1 mm². Each inlet can include a cross-sectional area between 0.25 mm² and 1.25 mm². In examples, each inlet can include a width, e.g., the width 208, greater than 0.5 mm. In examples, each inlet can include a width between 0.5 mm and 1.0 mm. In examples, each inlet can include a width between 0.25 mm and 1.25 mm. In examples, each inlet can include a width, e.g., the width 208, greater at least two-percent greater than an outer circumference of the catheter. In examples, each inlet can include a width greater than 0.5% the outer circumference of the catheter. In examples, each inlet can include a width between 0.5% and 2.5% the outer circumference of the catheter. In examples, each inlet can include a width that is less than 2.5% the outer circumference of the catheter. In example, each inlet length, e.g., length 210, can be less than 10% the outer circumference of the catheter. In examples, the length of the inlet can be between 10% and 20% the outer circumference of the catheter. In examples, the length of each inlet can be between 10% and 20% the outer circumference of the catheter.

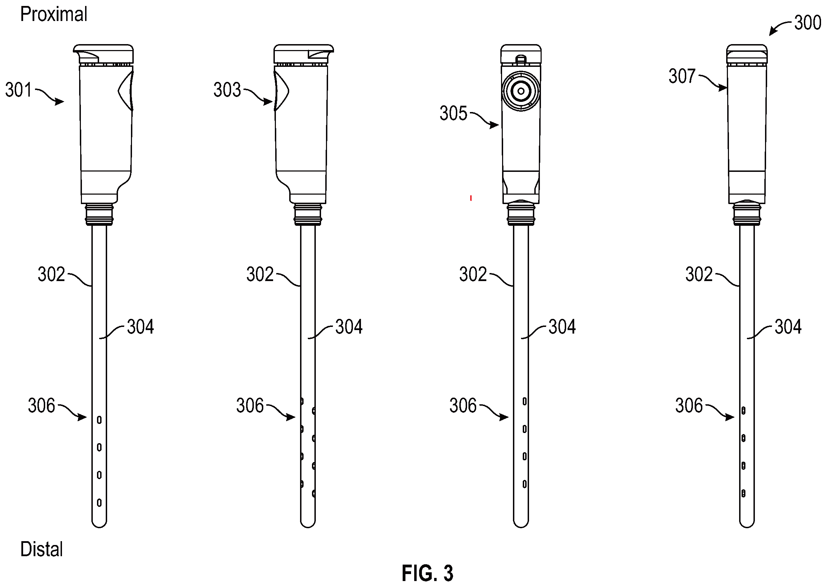

FIG. 3 illustrates an example of an intermittent catheter, e.g., a catheter 300, including, a rear view 301, a front view 303, a right side view 305, and a left side view 307. As shown in FIG. 3, the catheter 300 can be a configured for use by a user with a vagina and a length of the shaft 302 can be decreased to limit the overall length of the catheter 300 as compared to the shaft 102 and the overall length of the the catheter 100 (both in FIG. 1) for users with a penis. The decreased length of the shaft 202 can help reduce the likelihood that a clinician or user will incidentally hit, knock, or obstruct the catheter while the catheter 300 is inserted within the patient. As also shown in FIG. 3, the catheter 300 can include inlets 306 that can be short slots and can include multiple sets. Each set can include inlets that can be circumferentially aligned with the other inlets of the set and can be circumferentially offset from inlets of other sets of inlets.

The catheter 300 can also incorporate inlets 306 in the form of short slots, arranged in multiple sets. Each set of inlets can be circumferentially aligned with others in the same set, while being offset from inlets in other sets. The helical pattern of the inlets can provide improved drainage and reduced blockage risk. The slot-shaped design of the inlets 306 can enhance the catheter's ability to clear debris, tissue, or stones while still allowing efficient urine flow. By combining these features - a shorter shaft and optimized inlet design - the catheter 300 can offer a solution that addresses both the practical aspects of catheter use in healthcare settings and the fundamental challenges of efficient drainage and user comfort.

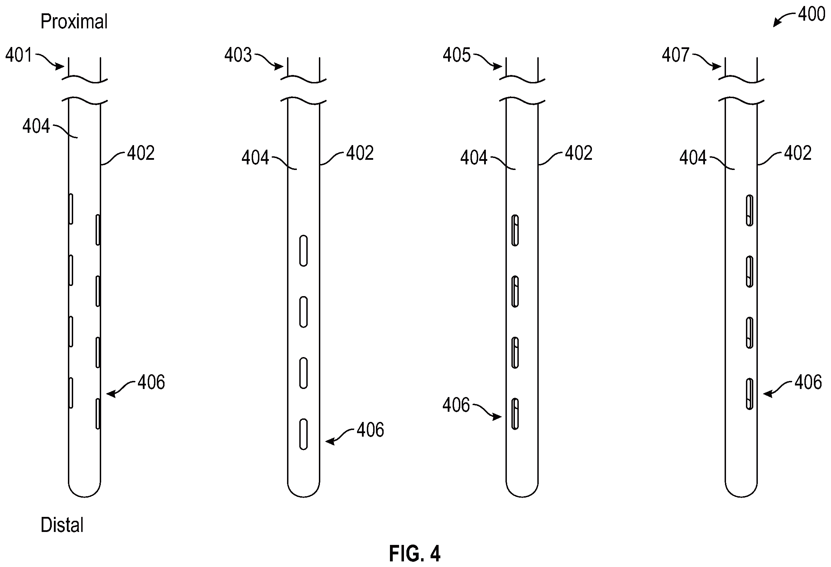

FIG. 4 illustrates an example of a distal end portion of an example of a catheter 400. The inlets 406 can include slots. The inlet 406 can include multiple sets of four inlets 406. Each set of the inlets 406 can be circumferentially aligned with inlets of the same set and circumferentially offset from inlets of other sets. As such, the inlets 406 can extend in a helical pattern. The slot shape of the inlets 406 can help clear debris, tissue, stones, or the like, and still permit urine or other fluid to flow through the inlets 406 and into the lumen 404.

In examples, each set of the inlets 406 can include two or more of the inlets 406. For example, each set can include three of the inlets 406, four of the inlets 406, five of the inlets 406, or six or more of the inlets 406. There can also be multiple sets, such as two, three, four, five, six, seven, eight, or more sets of the inlets 406. In examples, the total surface area of the inlets 406 can be greater than a cross-sectional area of the lumen 404. For example, the total surface area of the inlets 406 can be two or more times the cross-sectional area of the lumen 404.

In examples, the catheter 400 can include inlets 406 in the form of slots, arranged in multiple sets of four. In this example, each slot forming an inlet 406 is a rectangle with rounded opposing ends. Each set of inlets can be circumferentially aligned with others in the same set, while being offset from inlets in other sets. This configuration can create a helical pattern of inlets extending along the distal end portion. The slot shape of the inlets 406 can improve the catheter's ability to clear debris, tissue, and stones while maintaining efficient urine flow. By incorporating multiple sets of inlets, the design can increase the total inlet area, which can be greater than the cross-sectional area of the lumen 404. This increased inlet area can help ensure adequate drainage even if some inlets become partially blocked. The helical arrangement and slot shape can reduce the likelihood of complete blockage by reducing the likelihood of tissue or debris to obstructing all inlets simultaneously. Thus, the catheter 400 can provide more consistent and efficient bladder emptying and improved user comfort during catheter use.

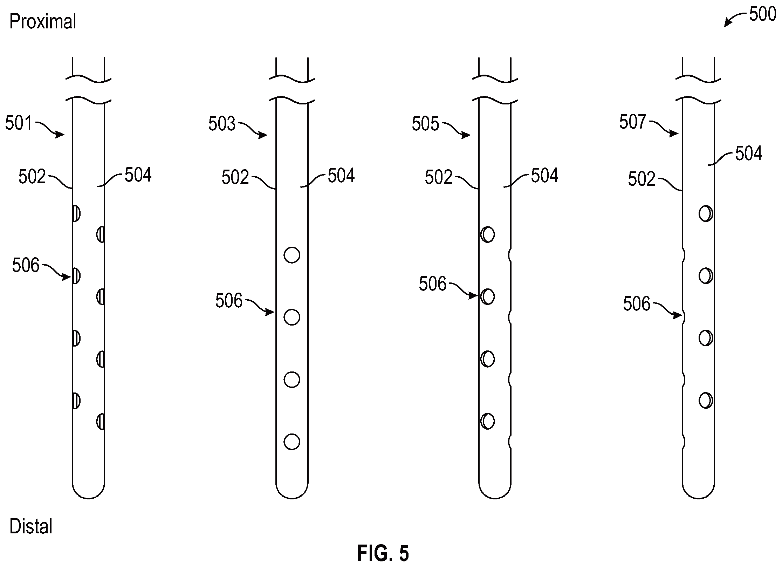

FIG. 5 illustrates an example of a distal end portion of an example of a catheter 500. The inlets 506 can include apertures, circular, or round openings in the shaft 502. The inlet 506 can include multiple sets of four inlets 506. Each set of inlets 506 can be circumferentially aligned with inlets of the same set and circumferentially offset from inlets of other sets. As such, the inlets 506 can extend in a helical pattern. The aperture shape of the inlets 406 can help clear debris, tissue, stones, or the like, and still permit urine or other fluid to flow through the inlets 506 and into the lumen 504.

In examples, each set of the inlets 506 can include two or more of the inlets 506. For example, each set can include three of the inlets 506, four of the inlets 506, five of the inlets 506, or six or more of the inlets 506. There can also be multiple sets, such as two, three, four, five, six, seven, eight, or more sets of the inlets 506. In examples, the total surface area of the inlets 506 can be greater than a cross-sectional area of the lumen 504. For example, the total surface area of the inlets 506 can be two or more times the cross-sectional area of the lumen 504.

In examples, the helical pattern of the inlets 506 can help reduce the risk of complete blockage, as it's less likely for tissue or debris to obstruct all inlets simultaneously. The circular shape of the inlets 506 can potentially offer a balance between efficient fluid flow and structural integrity of the shaft. By incorporating multiple sets of inlets, the design can increase the total inlet area, which can be greater than the cross-sectional area of the lumen 504. This increased inlet area can help ensure adequate drainage even if some inlets become partially obstructed. The aperture shape of the inlets 506 can also facilitate the passage of small debris or stones, potentially reducing the risk of catheter blockage. Thus, the catheter 500 can provide improved drainage efficiency and user comfort.

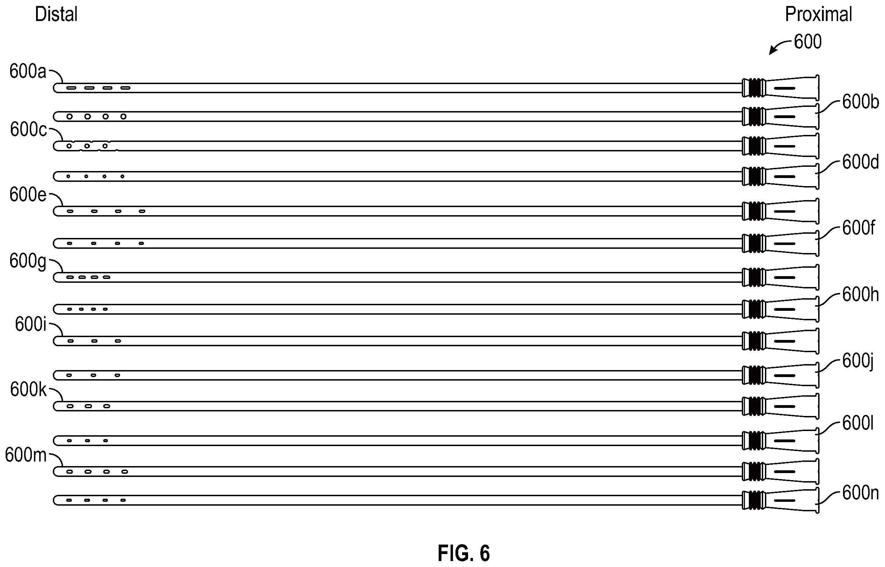

FIG. 6 illustrates examples of catheters 600. As shown in FIG. 6, each catheter of the catheters 600 can include different sizes, shapes, configurations, or the like of the inlets. For example, the first catheter 600a can include four slot-shaped inlets in each set of circumferentially aligned inlets. The second catheter 600b can include four round, circular, or aperture-shaped inlets for each set of circumferentially aligned inlets. The third catheter 600c can include three round, circular, or aperture-shaped inlets for each set of circumferentially aligned inlets. The fourth catheter 600d can include four small round, circular, or aperture-shaped inlets for each set of circumferentially aligned inlets. The fifth catheter 600e can include four slot-shaped inlets for each set of circumferentially aligned inlets. The sixth catheter 600f can include four small round, circular, or aperture-shaped inlets for each set of circumferentially aligned inlets. The seventh catheter 600g can include four closely spaced slot-shaped inlets for each set of circumferentially aligned inlets. The eight catheter 600h can include four closely spaced round, circular, or aperture-shaped inlets for each set of circumferentially aligned inlets. The ninth catheter 600i can include three large-spaced slot-shaped inlets for each set of circumferentially aligned inlets. The tenth catheter 600j can include three large-spaced circular, round, or aperture-shaped inlets for each set of circumferentially aligned inlets. The eleventh catheter 600k can include three slot-shaped inlets for each set of circumferentially aligned inlets. The twelfth catheter 600l can include three closely spaced small circular, round, or aperture-shaped inlets for each set of circumferentially aligned inlets. The thirteenth catheter 600m can include four slot-shaped inlets for each set of circumferentially aligned inlets. The fourteenth catheter 600n can include four large spaced small round, circular, or aperture-shaped inlets for each set of circumferentially aligned inlets.

Though these are examples of the catheters 600, any combination of inlets shown here can be used or combined into a single example of the catheters 600. For example, the size, spacing, shape, or area of the inlets can be altered for every inlet formed in the catheters 600. Thus, any combination of the shown inlets can be formed in one or more sets of multiple sets of inlets for the catheters 600.

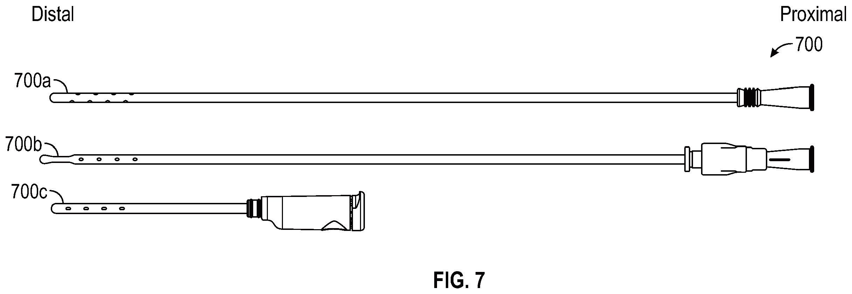

FIG. 7 illustrates examples of catheters 700. Each of the catheters 700 can include different length shaft or different adapters or funnels to connect the catheter to collection bags, waste fluid containers, a fluid collection system, or the like. In examples, the catheters 700 can be used near a toilet such that the catheters 700 is not connected to a fluid collection bag, and the urine can flow from the catheters 700 into the toilet. For example, the first catheter 700a can include a long shaft and a standard tube fitting to be able to fluidly connect the first catheter 700a to a fluid collection bag, container, system, or the like. In the example shown in first catheter 700a, the adapter can be coupled directly and at least partially permanently to the shaft of the first catheter 700a.

The second catheter 700b can include a distal end with a recessed portion. In examples, the recessed portion of the second catheter 700b can include a collapsed sleeve insertion aid to help the user insert the second catheter 700b. The recessed portion can be formed in the distal end and can have a diameter smaller than the rest of the shaft of the second catheter 700b. In examples, such as shown in FIG. 7, the recessed portion can be distal to the inlets formed in the shaft of the second catheter 700b. In some examples, the recessed portion can be formed with at least one inlet distal and at least one inlet proximal to the recessed portion. As shown in FIG. 7, the recessed portion can be slightly spaced proximally from the distal end of the second catheter 700b. In examples, the recessed portion can be formed at the distal end or can be further spaced proximally from the distal end of the second catheter 700b. In examples, the adapter can be removably coupled to the proximal end of the shaft, such that the shaft of the second catheter 700b can be at least partially inserted into the adapter during the setup or assembly of the second catheter 700b.

The third catheter 700c be configured for use by a user with a vagina and can include a shorter shaft than the first catheter 700a or second catheter 700b. As shown in FIG. 7, the adapter or funnel can help direct urine out of the third catheter 700c into a bag, container, fluid collection system, a toilet, or the like.

As discussed herein, the examples of the catheters 700 can be implemented into any of the catheters, e.g., the catheter 100, the catheter 200, the catheter 300, the catheter 400, the catheter 500, the catheters 600, or the catheters 700 with any combination of inlets, sets of inlets, or multiples of sets of inlets as discussed herein.

In examples, the examples of catheters illustrated in FIGS. 1-7 can collectively demonstrate various approaches to addressing common challenges in intermittent catheter design and use. These designs discussed herein can improve drainage efficiency, reduce the risk of blockage, enhance user comfort, and increase usability in clinical settings.

For example, the helical arrangement of inlets, as shown at least in FIGS. 1, 3, 4, and 5, can help decrease the probability of complete inlet blockage by tissue, debris, or stones. This configuration can promote more consistent and adequate drainage of the bladder. The various inlet shapes explored, including slots (at least shown in FIGS. 3 and 4) and circular apertures (at least shown in FIG. 5), can offer different benefits in terms of fluid flow and debris clearance. The size of the inlets can also help facilitate urine drainage from the bladder of the user. For example, the total area of the inlets can be greater than the cross-sectional area of the lumen to encourage urine flow within into the lumen of the catheter.

The optimization of inlet size and distribution, as detailed at least in FIG. 2, can demonstrate how adjusting width-to-length ratios and total inlet area can balance drainage efficiency with structural integrity of the catheter shaft. This approach can be seen implemented in various ways across the other figures.

Catheters for use by users with a vagina, as discussed at least in FIG. 3 can limit the extension of the catheter from the user. The shorter length of the shaft can aid in the removal of urine while the user is sitting on a toilet or squatting near a toilet. The shorter length shaft can also limit an amount the catheter extends from the patient to limit a likelihood of incidental contact with the catheter while inserted into the urethra of the vagina. Therefore, as discussed herein, the inlet design can be implemented across catheters for users including vaginas and users including penises and can effectively help alleviate or drain urine from within the bladder of the user.

Overall, these figures can present a comprehensive approach to intermittent catheter design, including catheters for users with a penis or users with a vagina, addressing key challenges such as drainage efficiency, blockage prevention, and user comfort. By incorporating these various design elements, future catheter designs can potentially offer improved performance and user experience in intermittent catheterization.

The following, non-limiting examples, detail certain aspects of the present subject matter to solve the challenges and provide the benefits discussed herein, among others.

Example 1 is an intermittent urinary catheter comprising: a shaft extending between a proximal end portion and a distal end portion, the shaft defining a lumen therethrough; and a plurality of inlets formed in the distal end portion, each inlet of the plurality of inlets in fluidic communication with the lumen, the plurality of inlets arranged in a helical pattern extending from a position adjacent a distal end portion of the shaft toward the proximal end portion.

In Example 2, the subject matter of Example 1 optionally includes wherein each inlet of the plurality of inlets extends circumferentially to define a width and extends longitudinally to define a length.

In Example 3, the subject matter of Example 2 optionally includes wherein each inlet of the plurality of inlets includes a width-to-length ratio of one to two.

In Example 4, the subject matter of any one or more of Examples 2-3 optionally include wherein each inlet of the plurality of inlets includes a width-to-length ratio between one to two and one to three.

In Example 5, the subject matter of any one or more of Examples 2-4 optionally include wherein each inlet of the plurality of inlets includes a width-to-length ratio between one to one and one to four.

In Example 6, the subject matter of any one or more of Examples 1-5 optionally include wherein a total inlet area of the plurality of inlets is greater than a cross-sectional area of the lumen.

In Example 7, the subject matter of any one or more of Examples 1-6 optionally include wherein a total inlet area of the plurality of inlets is at least twice a cross-sectional area of the lumen.

In Example 8, the subject matter of any one or more of Examples 1-7 optionally include wherein the plurality of inlets includes: a first set of inlets, each inlet of the first set of inlets is circumferentially aligned around the shaft with other inlets of the first set of inlets; a second set of inlets, each inlet of the second set of inlets is circumferentially aligned around the shaft with other inlets of the second set of inlets; and a third set of inlets, each inlet of the third set of inlets is circumferentially aligned around the shaft with other inlets of the third set of inlets.

In Example 9, the subject matter of Example 8 optionally includes wherein the first set of inlets and the second set of inlets are circumferentially offset and the second set of inlets and the third set of inlets are circumferentially offset to form the helical pattern of the plurality of inlets.

In Example 10, the subject matter of Example 9 optionally includes wherein each of the first set of inlets, the second set of inlets, and the third set of inlets includes four inlets, and wherein each set of inlets is circumferentially offset one hundred and twenty degrees from adjacent sets of inlets.

In Example 11, the subject matter of any one or more of Examples 9-10 optionally include wherein each of the first set of inlets, the second set of inlets, and the third set of inlets include three inlets, and wherein each set of inlets are circumferentially offset one hundred and twenty degrees from adjacent sets of inlets.

In Example 12, the subject matter of any one or more of Examples 9-11 optionally include wherein each inlet of the first set of inlets is longitudinally spaced from each inlet of the second set of inlets and each inlet of the third set of inlets, and wherein each inlet of the second set of inlets is longitudinally spaced from each inlet of the third set of inlets.

In Example 13, the subject matter of any one or more of Examples 8-12 optionally include a fourth set of inlets, each inlet of the fourth set of inlets is circumferentially aligned around the shaft with the other inlets of the fourth set of inlets.

In Example 14, the subject matter of Example 13 optionally includes wherein each of the first set of inlets, the second set of inlets, the third set of inlets, and the fourth set of inlets include three inlets, and wherein each set of inlets are circumferentially offset ninety degrees from adjacent sets of inlets.

In Example 15, the subject matter of any one or more of Examples 1-14 optionally include wherein each inlet of the plurality of inlets are slots extending from the distal end portion toward the proximal end portion of the shaft.

Example 16 is an intermittent urinary catheter comprising: a shaft extending between a proximal end portion and a distal end portion, the shaft defining a lumen therethrough; and a plurality of inlets formed in the distal end portion, each inlet of the plurality of inlets in fluidic communication with the lumen, a total area of the plurality of inlets is greater than a cross-sectional area of the lumen.

In Example 17, the subject matter of Example 16 optionally includes wherein the plurality of inlets extend longitudinally form the distal end portion toward the proximal end portion in a helical pattern.

In Example 18, the subject matter of any one or more of Examples 16-17 optionally include wherein the total area of the plurality of inlets is at least twice the cross-sectional area of the lumen.

In Example 19, the subject matter of any one or more of Examples 16-18 optionally include wherein each inlet of the plurality of inlets are slots extending from the distal end portion toward the proximal end portion of the shaft, and wherein each inlet is longitudinally spaced at least a distance equal to a length of the slot from adjacent inlets.

In Example 20, the subject matter of any one or more of Examples 16-19 optionally include wherein each inlet of the plurality of inlets includes a slot or a circular profile.

Example 21 includes a method, device, system, or the like, including any element or portion of an element from any one or more of Examples 1-20.

The above detailed description includes references to the accompanying drawings, which form a part of the detailed description. The drawings show, by way of illustration, specific examples that may be practiced. These embodiments are also referred to herein as "examples." Such examples may include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

All publications, patents, and patent documents referred to in this document are incorporated by reference herein in their entirety, as though individually incorporated by reference. In the event of inconsistent usages between this document and those documents so incorporated by reference, the usage in the incorporated reference(s) should be considered supplementary to that of this document; for irreconcilable inconsistencies, the usage in this document controls.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In the appended claims, the terms "including" and "in which" are used as the plain-English equivalents of the respective terms "comprising" and "wherein." Also, in the following claims, the terms "including" and "comprising" are open-ended, that is, a system, device, article, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The term "about," as used herein, means approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10%. In one aspect, the term "about" means plus or minus 10% of the numerical value of the number with which it is being used. Therefore, about 50% means in the range of 45%-55%. Numerical ranges recited herein by endpoints include all numbers and fractions subsumed within that range (e.g., 1 to 5 includes 1, 1.5, 2, 2.75, 3, 3.90, 4, 4.24, and 5). Similarly, numerical ranges recited herein by endpoints include subranges subsumed within that range (e.g., 1 to 5 includes 1-1.5, 1.5-2, 2-2.75, 2.75-3, 3-3.90, 3.90-4, 4-4.24, 4.24-5, 2-5, 3-5, 1-4, and 2-4). It is also to be understood that all numbers and fractions thereof are presumed to be modified by the term "about."

The above description is intended to be illustrative, and not restrictive. For example, the above-described examples (or one or more aspects thereof) may be used in combination with each other. Other examples may be used, such as by one of ordinary skill in the art upon reviewing the above description. The Abstract is to allow the reader to quickly ascertain the nature of the technical disclosure and is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description, with each claim standing on its own as a separate embodiment. The scope of the examples should be determined with reference to the appended claims, along with the full scope of equivalents to which such claims are entitled.

## Claims

1. An intermittent urinary catheter comprising:
a shaft extending between a proximal end portion and a distal end portion, the shaft defining a lumen therethrough; and
a plurality of inlets formed in the distal end portion, each inlet of the plurality of inlets in fluidic communication with the lumen, the plurality of inlets arranged in a helical pattern extending from a position adjacent a distal end portion of the shaft toward the proximal end portion.

2. The intermittent urinary catheter of claim 1, wherein each inlet of the plurality of inlets extends circumferentially to define a width and extends longitudinally to define a length.

3. The intermittent urinary catheter of claim 2, wherein each inlet of the plurality of inlets includes a width-to-length ratio of 1:2.

4. The intermittent urinary catheter of claim 2, wherein each inlet of the plurality of inlets includes a width-to-length ratio between 1:2 and 1:3.

5. The intermittent urinary catheter of claim 2, wherein each inlet of the plurality of inlets includes a width-to-length ratio between 1:1 and 1:4.

6. The intermittent urinary catheter of claim 1, wherein a total inlet area of the plurality of inlets is greater than a cross-sectional area of the lumen.

7. The intermittent urinary catheter of claim 1, wherein a total inlet area of the plurality of inlets is at least twice a cross-sectional area of the lumen.

8. The intermittent urinary catheter of claim 1, wherein the plurality of inlets includes:
a first set of inlets, each inlet of the first set of inlets is circumferentially aligned around the shaft with other inlets of the first set of inlets;
a second set of inlets, each inlet of the second set of inlets is circumferentially aligned around the shaft with other inlets of the second set of inlets; and
a third set of inlets, each inlet of the third set of inlets is circumferentially aligned around the shaft with other inlets of the third set of inlets.

9. The intermittent urinary catheter of claim 8, wherein the first set of inlets and the second set of inlets are circumferentially offset and the second set of inlets and the third set of inlets are circumferentially offset to form the helical pattern of the plurality of inlets.

10. The intermittent urinary catheter of claim 9, wherein each of the first set of inlets, the second set of inlets, and the third set of inlets include four inlets, and wherein each set of inlets are circumferentially offset 120 degrees from adjacent sets of inlets.

11. The intermittent urinary catheter of claim 9, wherein each of the first set of inlets, the second set of inlets, and the third set of inlets include three inlets, and wherein each set of inlets are circumferentially offset 120 degrees from adjacent sets of inlets.

12. The intermittent urinary catheter of claim 9, wherein each inlet of the first set of inlets is longitudinally spaced from each inlet of the second set of inlets and each inlet of the third set of inlets, and wherein each inlet of the second set of inlets is longitudinally spaced from each inlet of the third set of inlets.

13. The intermittent urinary catheter of claim 8, further comprising:
a fourth set of inlets, each inlet of the fourth set of inlets is circumferentially aligned around the shaft with the other inlets of the fourth set of inlets.

14. The intermittent urinary catheter of claim 13, wherein each of the first set of inlets, the second set of inlets, the third set of inlets, and the fourth set of inlets include three inlets, and wherein each set of inlets are circumferentially offset 90 degrees from adjacent sets of inlets.

15. The intermittent urinary catheter of claim 1, wherein each inlet of the plurality of inlets are slots extending from the distal end portion toward the proximal end portion of the shaft.
